# FASCICULE DE BREVET EUROPEEN

(11) **EP 1 610 726 B1**
(45) Date de publication et mention de la délivrance du brevet: **13.05.2009**
(21) Numéro de dépôt: 04742371.0
(22) Date de dépôt: 26.03.2004
(51) Int. Cl.: A61F 2/16

(54) **LENTILLE INTRAOCULAIRE ET SON INJECTEUR**
INTRAOKULARLINSE UND INJEKTOR DAFÜR
INTRAOCULAR LENS AND INJECTOR FOR THE SAME

(30) Priorité: 01.04.2003 FR 0304019
(43) Date de publication de la demande: 04.01.2006
(73) Titulaire: Laboratoire de Contactologie Appliquée - LCA, 28000 Chartres (FR)
(72) Inventeur: VINCENT, Patrice, F-28130 Mevoisins (FR)
(74) Mandataire: CAPRI
(86) Numéro de dépôt international: PCT/FR2004/000767
(87) Numéro de publication internationale: WO 2004/089251

(56) Documents cités:
- WO-A-00/49974
- WO-A-00/53124
- WO-A-83/00998
- US-A- 4 242 762
- US-A- 5 074 877
- US-B1- 6 461 384

## Description

La présente invention concerne une lentille intraoculaire injectable et un injecteur pour distribuer une telle lentille.

De manière connue, une lentille intraoculaire est destinée à remplacer le cristallin d'un patient opéré de la cataracte. L'haptique de la lentille intraoculaire est la partie qui se situe à l'extérieur de l'optique et qui sert à maintenir la lentille en position à l'intérieur de l'oeil du patient. De manière habituelle, l'haptique inférieure est celle qui est introduite en premier, sachant que l'incision est le plus souvent située du côté frontal, le chirurgien étant durant l'intervention placé derrière la tête du patient couché. L'haptique supérieure qui est placée en dernier, reste la plus proche de l'incision.

Le concept de l'injecteur est apparu avec celui des lentilles intraoculaires réalisées en matériau souple, déformable mécaniquement. Ce développement répond à l'utilisation pour la chirurgie de la cataracte de petites incisions, dont la largeur est souvent limitée à environ 3 mm. L'embout de l'injecteur devrait pouvoir passer à travers cette incision sans qu'il soit besoin de l'élargir.

Il existe de nombreux modèles d'injecteurs pour lentille intraoculaire. Dans la plupart des cas, la lentille intraoculaire est pliée par un dispositif annexe tel qu'une cartouche à volet, un tiroir ou une nervure d'appui, de telle sorte que le rôle du piston se résume à la poussée finale à travers l'embout.

L'embout cylindrique de l'injecteur est de diamètre moyen interne plus petit (1,5 à 4 mm) que le diamètre optique de la lentille intraoculaire (5 à 7 mm). L'idée de faire passer cette lentille dans une section se réduisant progressivement, donc d'allure plus ou moins conique a, déjà été divulguée dans des brevets antérieurs, et notamment dans les documents WO 00/49974 et WO 02/00870.

Un autre exemple de l'art antérieur est décrit dans WO-83/00998.

Une des difficultés majeures de cette technique consiste à obtenir que la lentille conserve son alignement dans le système, durant toute la phase de la compression diamétrale de l'optique.

En particulier, la forme environ circulaire de l'optique peut entraîner un déplacement transversal, et donc une perte d'alignement axial de la lentille.

D'autre part, l'haptique inférieure de la lentille, disposée en aval de l'optique dans le sens du distributeur de la lentille, risque de frotter prématurément contre la paroi interne comique de l'injecteur, ce qui peut avoir pour effet de déstabiliser la lentille ou de replier l'haptique inférieure sur l'optique, compromettant ainsi l'éjection de cette lentille.

La présente invention a pour but de fournir une lentille intraoculaire et un injecteur associé qui ne reproduisent pas les inconvénients susmentionnés.

La présente invention a notamment pour but de fournir une telle lentille et un tel injecteur qui fonctionnent de manière sûre et fiable, sans risque de blocage de la lentille dans l'injecteur lors de sa distribution.

La présente invention a aussi pour but de fournir une telle lentille et un tel injecteur qui soient simples et peu coûteux à fabriquer et à réaliser.

La présente invention a donc pour objet une lentille intraoculaire injectable en matière souple, ladite lentille comportant une optique de forme environ circulaire, ladite lentille comportant deux méplats latéraux prévus sur deux bords opposés de l'optique, lesdits méplats formant un angle l'un par rapport à l'autre.

Avantageusement, ladite optique est symétrique par rapport à un axe longitudinal central de la lentille.

Avantageusement, lesdits méplats ont chacun une longueur d'au moins 0,5 millimètre, de préférence d'environ 1 millimètre.

Avantageusement, ladite lentille comporte une haptique inférieure de dimension transversale inférieure à celle de l'optique.

Avantageusement, ladite lentille comporte une haptique supérieure.

Avantageusement, ladite haptique supérieure comporte deux éléments latéraux.

La présente invention a aussi pour objet un injecteur de lentille intraoculaire comportant une partie de corps principale cylindrique et une partie de corps conique débouchant dans une partie de distribution de dimension réduite, ledit injecteur comportant une lentille telle que définie ci-dessus.

Avantageusement, avant distribution, ladite lentille est disposée sans être déformée dans ledit injecteur.

Avantageusement, l'angle formé par lesdits méplats de la lentille correspond à l'angle formé par la paroi de la partie conique de l'injecteur.

Avantageusement, avant distribution, l'haptique inférieure de la lentille est espacée de la paroi de la partie de corps conique d'au moins 0,2 millimètres, de préférence d'environ 0,5 millimètres.

Avantageusement, ledit injecteur comprend un piston adapté à déplacer ladite lentille de la partie de corps cylindrique, en passant par la partie de corps conique, vers la partie de distribution.

Avantageusement, ledit piston coopère avec ladite haptique supérieure et/ou avec ladite optique de la lentille.

D'autres caractéristiques et avantages de la présente invention apparaîtront plus clairement au cours de la description détaillée suivante d'un mode de réalisation de la présente invention, faite en référence aux dessins joints, donnés à titre d'exemples non limitatifs, et sur lesquels,
la figure 1 est une vue schématique en section d'une lentille intraoculaire selon un mode de réalisation de la présente invention ; et
les figures 2 à 4 représentent des vues schématiques de l'injecteur selon un mode de réalisation de la présente invention, respectivement en début, en cours et en fin de distribution de la lentille intraoculaire de la figure 1.

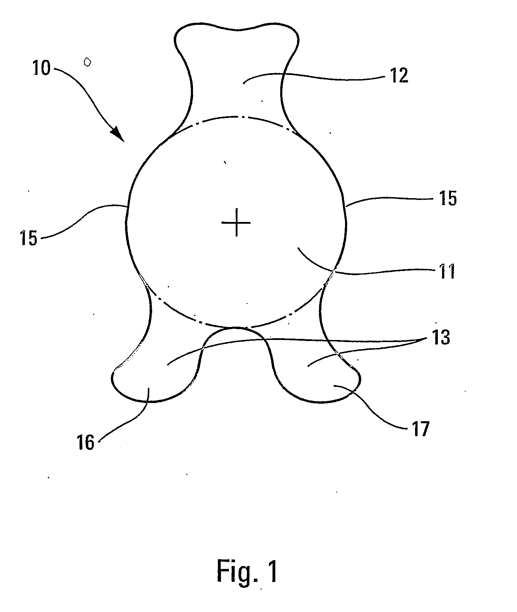

La figure 1 montre schématiquement une lentille intraoculaire selon l'invention.

La lentille intraoculaire 10 comporte une optique 11, de préférence de forme environ circulaire, adaptée à se plier dès qu'elle est comprimée selon son diamètre. La lentille 10 comporte avantageusement une haptique inférieure 12, qui est disposée en aval dans le sens du déplacement de la lentille à l'intérieur du corps de l'injecteur, et/ou une haptique supérieure 13, qui peut coopérer avec ledit piston 24. Cette haptique supérieure 13 peut comporter deux éléments latéraux 16, 17, avantageusement symétriques.

Selon l'invention, l'optique 11 de la lentille comporte deux méplats 15 situés sur le bord de l'optique, sur deux côtés opposés de celle-ci. Comme visible sur la figure 1, ces méplats 15 forment un angle l'un par rapport à l'autre. De préférence, la lentille, et notamment l'optique 11, sont symétriques par rapport à l'axe longitudinal central de la lentille. La longueur minimale de chaque méplat 15 est de 0,5 millimètre, et de préférence, cette longueur est d'environ 1 millimètre.

Avantageusement, cette lentille 10 est réalisée de manière monobloc en matière souple. De préférence, la dimension transversale de l'haptique inférieure 12 est inférieure à celle de l'optique 11. Le but est d'éviter que l'haptique inférieure 12 n'amorce son pliage dans l'injecteur avant l'optique 11, ce qui risquerait de bloquer la distribution de la lentille. A titre d'exemple, l'optique 11 peut avoir une largeur de 5,4 millimètres et l'haptique inférieure 12 de 3 millimètres.

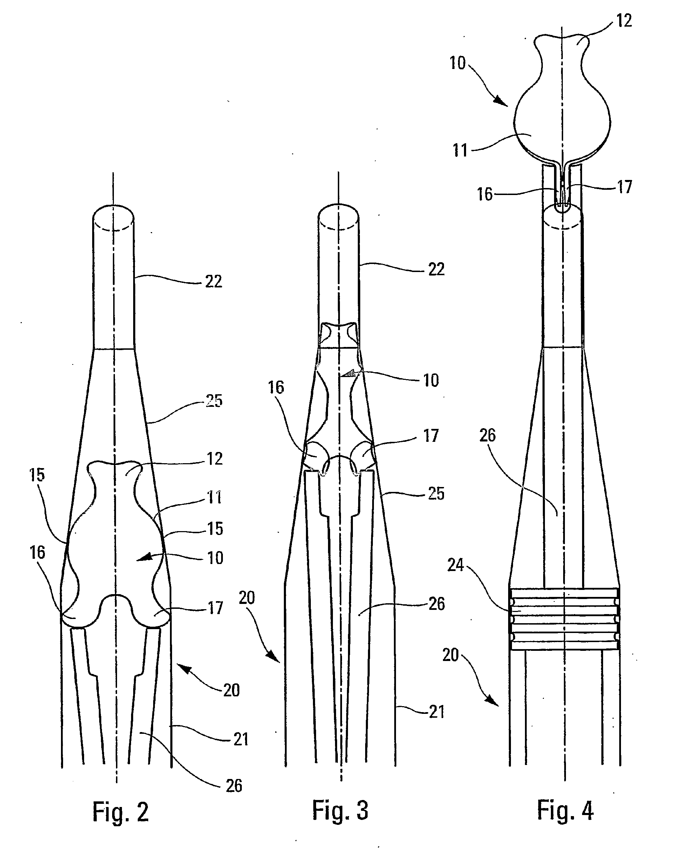

En référence aux figures 2 à 4, il est représenté un mode de réalisation particulier de l'injecteur 20 selon la présente invention. Dans ce mode de réalisation, l'injecteur comporte un corps constitué d'une partie principale 21 cylindrique, d'une partie de distribution 22 cylindrique, qui est de section transversale inférieure à ladite partie principale 21, et d'une partie de corps conique 25 qui relie ladite partie principale 21 à ladite partie de distribution 22. La conicité de la partie conique 25, à savoir l'angle formé par la paroi par rapport à l'axe longitudinal central de l'injecteur correspond avantageusement à l'angle des méplats de la lentille. La partie principale 21 est adaptée à contenir la lentille 10 non déformée, comme représenté sur la figure 2.

Les documents WO 00/49974 et WO 02/00870 sont cités ici à titre de référence en ce qui concerne la forme et le fonctionnement de l'injecteur 20.

Le piston 24 peut comporter un ou plusieurs brins 26, notamment deux dans l'exemple représenté. Ces brins 26 peuvent se rapprocher l'un de l'autre lorsque le piston 24 coulisse dans la partie conique 25.

Au départ, la lentille 10 est disposée de telle sorte que les deux brins 26 du piston 24 sont chacun vis-à-vis d'un élément latéral respectif 16, 17 de l'haptique supérieure 13. L'optique 11 est juste guidée lorsque le piston est actionné et l'haptique inférieure 12 qui est étroite est engagée sans contact dans la partie conique 25 de l'injecteur 20.

Lorsque l'on exerce une pression sur le piston 24, la lentille 10 est tout d'abord translatée sans déformation, jusqu'à ce que les deux méplats 15 de l'optique 11 arrivent au contact de la partie conique 25. En continuant la pression sur le piston 20, l'optique 11 amorce son pliage, la paroi de la partie conique 25 guidant les méplats 15 pour maintenir l'alignement axial de la lentille 10. L'optique 11 en cours de pliage est presque roulée tout en étant en contact avec la paroi de la partie conique 25. L'haptique inférieure 12 amorce ensuite son pliage, lorsqu'elle arrive en contact avec la partie conique 25 en suivant le mouvement de l'optique 11. La dimension radiale inférieure de l'haptique inférieure 12 évite que celle-ci ne se plie avant l'optique 11.

Après le pliage, la lentille 10 est poussée dans la partie de distribution 22.

En fin de poussée, représentée sur la figure 4, l'optique 11 s'est redéployée à l'intérieur de l'oeil du patient, et les éléments latéraux 16, 17 de l'haptique supérieure 13 sont plaqués l'un contre l'autre.

La présente invention a été décrite ci-dessus en référence à un mode de réalisation avantageux de celle-ci, mais il est clair qu'elle ne se limite pas à ce mode de réalisation. En particulier, la forme des haptiques peut être quelconque, et ces haptiques ne sont pas nécessairement réalisées de manière monobloc avec l'optique de la lentille. La forme de l'haptique supérieure 13 représentée sur le dessin est particulièrement adaptée à la transmission de la poussée par un piston multibrins. Bien entendu, le piston peut être quelconque et pourrait coopérer avec l'haptique supérieure, avec l'optique, ou même avec les deux. D'autre part, des lentilles comportant des haptiques à anses rapportées ou des lentilles monoblocs ayant des formes différentes, par exemples des anses en forme de C, des navettes, des haptiques à quatre points d'appui, ou similaires sont également couvertes par la présente invention.

D'autres modifications peuvent être apportées par l'homme du métier sans sortir du cadre de la présente invention, telle que définie par les revendications annexées.

## Revendications

1. Lentille intraoculaire injectable (10) en matière souple, ladite lentille (10) comportant une optique (11) de forme environ circulaire ladite lentille (10) comporte deux méplats latéraux (15) prévus sur deux bords opposés de l'optique (11), **caractérisée en ce que** lesdits méplats (15) formant un angle l'un par rapport à l'autre.

2. Lentille selon la revendication 1, dans laquelle ladite optique (11) est symétrique par rapport à un axe longitudinal central de la lentille (10).

3. Lentille selon la revendication 1 ou 2, dans laquelle lesdits méplats (15) ont chacun une longueur d'au moins 0,5 millimètre, de préférence d'environ 1 millimètre.

4. Lentille selon l'une quelconque des revendications précédentes, dans laquelle ladite lentille (10) comporte une haptique inférieure (12) de dimension transversale inférieure à celle de l'optique (11). -

5. Lentille selon l'une quelconque des revendications précédentes, dans laquelle ladite lentille (10) comporte une haptique supérieure (13).

6. Lentille selon la revendication 5, dans laquelle ladite haptique supérieure (13) comporte deux éléments latéraux (16,17).

7. Ensemble comportant un injecteur (20) de lentille intraoculaire et une lentille intraoculaire (10), **caractérisé en ce que** ledit injecteur (20) comporte une partie de corps principale cylindrique (21) et une partie de corps conique (25) débouchant dans une partie de distribution (22) de dimension réduite, et **en ce que** ladite lentille (10) est réalisée selon l'une quelconque des revendications précédentes.

8. Ensemble selon la revendication 7, dans lequel, avant distribution, ladite lentille (10) est disposée sans être déformée dans ledit injecteur.

9. Ensemble selon la revendication 7 ou 8, dans lequel l'angle formé par lesdits méplats (15) de la lentille (10) correspond à l'angle formé par la paroi de la partie conique (25) de l'injecteur (20).

10. Ensemble selon l'une quelconque des revendications 7 à 9, dans lequel, avant distribution, l'haptique inférieure (12) de la lentille (10) est espacée de la paroi de la partie de corps conique (25) d'au moins 0,2 millimètres, de préférence d'environ 0,5 millimètres.

11. Ensemble selon l'une quelconque des revendications 7 à 10, dans lequel ledit injecteur (20) comprend un piston (24) adapté à déplacer ladite lentille (10) de la partie de corps cylindrique (21), en passant par la partie de corps conique (25), vers la partie de distribution (22).

12. Ensemble selon la revendication 11, dans lequel ledit piston (24) coopère avec ladite haptique supérieure (13) et/ou avec ladite optique (11) de la lentille (10).

## Claims

1. An injectable intraocular lens (10) made of flexible material, said lens (10) having an optical portion (11) that is of approximately circular shape, said injectable intraocular lens (10) having two side flats (15) provided on opposite edges of the optical portion (11), **characterized in that** said flats (15) form an angle relative to each other.

2. A lens according to claim 1, in which said optical portion (11) is symmetrical about a central longitudinal axis of the lens (10).

3. A lens according to claim 1 or claim 2, in which each of said flats (15) has a length of at least 0.5 mm, and preferably of about 1 mm.

4. A lens according to any preceding claim, in which said lens (10) has a lower haptic (12) of transverse dimension smaller than the transverse dimension of the optical portion (11).

5. A lens according to any preceding claim, in which said lens (10) has an upper haptic (13).

6. A lens according to claim 5, in which said upper haptic (13) has two side elements (16, 17).

7. An assembly comprising an intraocular lens injector (20) and an intraocular lens (10), said assembly being **characterized in that** said injector (20) has a cylindrical main body (21) and a conical body portion (25) opening out into a dispensing portion (22) of small size, and **in that** said lens (10) is made in accordance with any preceding claim.

8. An assembly according to claim 7, in which, prior to being dispensed, said lens (10) is disposed in said injector without being deformed.

9. An assembly according to claim 7 or claim 8, in which the angle formed by said flats (15) of the lens (10) corresponds to the angle formed by the wall of the conical portion (25) of the injector (20).

10. An assembly according to any one of claims 7 to 9, in which, prior to dispensing, the lower haptic (12) of the lens (10) is spaced apart from the wall of the conical body portion (25) by at least 0.2 mm, and preferably about 0.5 mm.

11. An assembly according to any one of claims 7 to 10, in which said injector (20) has a piston (24) adapted to move said lens (10) from the cylindrical body portion (21), through the conical body portion (25), to the dispensing portion (22).

12. An assembly according to claim 11, in which said piston (24) co-operates with said upper haptic (13) and/or with said optical portion (11) of the lens (10).

## Patentansprüche

1. Durch Spritzen herstellbare Intraokularlinse (10) aus biegsamem Material, wobei die Linse (10) eine Optik (11) in etwa kreisförmiger Form aufweist, wobei die Linse (10) zwei seitliche Abflachungen (15) aufweist, welche an zwei gegenüberliegenden Rändern der Optik (11) vorgesehen sind, **dadurch gekennzeichnet, dass** die Abflachungen (15) in Bezug aufeinander einen Winkel bilden.

2. Linse nach Anspruch 1, wobei die Optik (11) in Bezug auf die Längsmittenachse der Linse (10) symmetrisch ist.

3. Linse nach Anspruch 1 oder 2, wobei die Abflachungen (15) jeweils eine Länge von zumindest 0,5 mm, bevorzugt von ungefähr 1 mm, aufweisen.

4. Linse nach einem der vorangehenden Ansprüche, wobei die Linse (10) eine haptische Unterseite (12) einer Querabmessung aufweist, die kleiner ist als diejenige der Optik (11).

5. Linse nach einem der vorangehenden Ansprüche, wobei die Linse (10) eine obere Haptik (13) aufweist.

6. Linse nach Anspruch 5, wobei die obere Haptik (13) zwei seitliche Elemente (16, 17) aufweist.

7. Einheit, aufweisend einen Injektor (20) für eine Intraokularlinse und eine Intraokularlinse (10), **dadurch gekennzeichnet, dass** der Injektor (20) einen zylindrischen Hauptkörperteil (21) und einen konischen Körperteil (25) aufweist, der in einem Abgabeteil (22) verlängerter Abmessung ausmündet, und dass die Linse (10) nach einem der vorangehenden Ansprüche hergestellt ist.

8. Einheit nach Anspruch 7, wobei die Linse (10) vor der Abgabe, ohne verformt zu werden, in dem Injektor angeordnet ist.

9. Einheit nach Anspruch 7 oder 8, wobei der durch die Abflachungen (15) der Linse (10) gebildete Winkel einem Winkel entspricht, der durch die Wandung des konischen Teils (25) des Injektors (20) gebildet ist.

10. Einheit nach einem der Ansprüche 7 bis 9, wobei die untere Haptik (12) der Linse (10) vor der Abgabe von der Wandung des konischen Körperteils (25) zumindest 0,2 mm, bevorzugt ungefähr 0,5 mm, beabstandet ist.

11. Einheit nach einem der Ansprüche 7 bis 10, wobei der Injektor (20) einen Kolben (24) aufweist, der dazu ausgelegt ist, die Linse (10) des zylindrischen Körperteils (21) en passant durch den konischen Körper (25) in Richtung auf den Abgabeteil (22) zu verschieben.

12. Einheit nach Anspruch 11, wobei der Kolben (24) mit der oberen Haptik (13) und/oder mit der Optik (11) der Linse (10) zusammenwirkt.
